# EUROPEAN PATENT APPLICATION

(11) **EP 1 552 741 A1**
(43) Date of publication of application: **13.07.2005**
(21) Application number: 04290066.2
(22) Date of filing: 09.01.2004
(51) Int. Cl.: A01K 67/027, A61K 49/00, G01N 33/50, C12N 5/06

(54) **Non-human mammal model comprising heterologous nucleated cells-use for screening compounds**

(71) Applicant: INSTITUT PASTEUR, 75724 Paris Cédex 15 (FR)
(72) Inventor: Druilhe, Pierre, 75015 Paris (FR); Badell-Ocando, Edgar, 75013 Paris (FR); Scott-Algara, Daniel, 94500 Champigny/Marne (FR); Hez, Stéphanie, 75015 Paris (FR)
(74) Representative: Desaix, Anne

(57) **Abstract**

The invention relates to a method of making a non-human mammal model comprising:
a. implanting, into an immunocompromised non-human mammal host, heterologous nucleated cells previously bound to a biocompatible support,
b. controlling non-adaptive defences of the non-human mammal host,
c. recovering a non-human mammal model harbouring settled heterologous nucleated cells capable of maintaining, differentiating and growing.

The invention also relates to a non-human mammal model which is an immunocompromised non-human mammal host implanted with a support comprising heterologous nucleated cells settled thereon, and which non-adaptive defences are controlled to enable the heterologous nucleated cells of said implanted support to maintain, differentiate and grow.

## Description

### FIELD OF THE INVENTION

The present invention provides a method of making a non-human mammal model comprising heterologous nucleated cells. The invention also discloses a non-human mammal model and a tissue matrix derived from such model. The invention also relates to applications in pathogen studies having recourse to said model, including for screening compounds or assessing efficacy of compounds in the treatment of pathogen infections or detrimental effects resulting from said infection. The invention also concerns the use of said model to evaluate the interest of compounds in treatment of patients.

The non-human mammal model comprising heterologous nucleated cells can further be useful for the study of metabolism of said cells, when said cells are submitted to contact with various agents including drug compounds or drug candidates.

### BACKGROUND OF THE INVENTION

Disease-causing pathogens include microorganisms encompassing viruses, bacteria, fungi or parasites. Other pathogens can be substances inducing or favoring toxic or detrimental reactions to emerge or to spread in hosts, said substances including components derived from microorganisms or produced by the same or can be molecules having a different origin. Pathogen infections in humans, sometimes leading to premature death, have been controlled to some extent in industrialized countries in the last decades due to a better comprehension of pathogen life cycle and to the design and availability of new drugs including vaccines. However, known pathogens keep on infecting people in various regions, whereas in other situations, resistance strains to existing drugs have occurred or new pathogens emerge. The needs therefore remain for the design or the identification of efficient drugs against pathogens or against their detrimental consequences in hosts and for the study of mechanisms of infection of such pathogens.

The study of pathogens can be performed both on *in vitro* or *in vivo* models. *In vitro* models, such as cell cultures, are easy to maintain at a reasonable price. However, culture cells are not always receptive to pathogens, and if they are, they do not sustain the infection for a sufficient time period enabling the study of the pathogen life cycle. Moreover, primary cultures are not differentiated enough to express markers and to secrete molecules. Finally, they are not integrated in an environment comparable to the environment offered by a live organism and consequently lack interactions with other biological systems operating *in vivo* and particularly with the immune system. As such, cellular cultures do not represent a sufficient model to study the various interactions between the pathogen and the cell in a manner which would mimic *in vivo* interactions.

*In vivo* models often represent more relevant models than cultured cells; experiments generally are carried on mammals and particularly on mice, but also on primates. Mouse models have a lot of advantages such as being cost efficient, easy to reproduce and to manipulate. However, many pathogens cannot develop in such a host because of their restricted tropism. Besides, biological mechanisms in mice are different in many respects from those observed in human and results obtained in mice can sometimes hardly be transposed to human. To overcome such problems, experiments are performed on primates where the mechanisms of infection are more or less the same as in human, at least in higher primates such as chimpanzees. But the limited availability of these primates, the economical and ethical considerations underlying their use and the difficulty to handle them in most laboratories severely restrict their use for such purposes.

A particular group of diseases, concerned by these restrictions, are human liver diseases, such as hepatitis and malaria for which yearly cumulative mortality is close to 10 millions people. The infection caused by 3 majors pathogens, HBV (hepatitis B virus), HCV (hepatitis C virus) and *Plasmodium falciparum* for malaria, can be fought by different treatments: preventive vaccine or antiviral therapy for HBV and antiviral therapy for HCV and malaria. However, some patients do not respond to treatment and resistant strains of said pathogens are increasing both in prevalence and degree of resistance. The development of pathogen studies and new drugs is hampered by the difficulty to establish *in vitro* and *in vivo* relevant models.

The narrow host range of these pathogens prevents their efficient study in most *in vitro* models. For example, only fully functional hepatocytes of primary cultures are susceptible to *Plasmodium falciparum,* but after 1 to 3 weeks of cultivation, these cultures become refractory when phenotypic changes, i.e., de-differentiation, occurs (Fraslin, EMBO J, 1985 and Guguen-guillouzou, cytotechnology, 1993). Even the most differentiated hepatoma, such as HepG2-A16 or BC2 which share 99% homology with primary hepatocytes in terms of secreted protein do not sustain *Plasmodium falciparum* maturation (Hollingdale Am J Trop Med Hyg, 1985 and Druilhe, in malaria 1998).

Whereas mammal models comprising tumour cells have been described, few mammal models comprising non-infected, non-tumoral human cells are available. The need in such models or in improved *in vivo* models, reproducing to a certain extent human cell conditions is important not only for the study of infectious diseases as explained above, but also for the study of non infectious diseases, such as genetic or environmental diseases, or more generally for the study of the metabolism of compounds of human cells embedded in the animal model.

Moreover, *in vivo* animal models would be useful to perform screening activity of compounds on organisms, especially for testing effects of new drugs on live organisms. In this respect, a non-human mammal according to the invention which comprises functional human cells, allows the study of metabolic pathways following administration of compounds. Due to differences existing in metabolic pathways between human patients and animal models usually used for screening it appears that the effects of a compound on a human biological system can sometimes be ascertained in clinical trials only. Thus, the availability of such models would enable to increase screening efficiency and thus select compounds of interest for clinical trials, in a more appropriate manner.

*In vivo* models which have been prepared over a ten-year period, offering the potential to store human healthy and infected cells *in vivo* but still presenting drawbacks which harm their effective use.

For example, in order to study the stage known as late stage of *Plasmodium falciparum* cycle, *i.e.* the hepatic or exoerythrocytic (EE) stage, human heptocytes have been transplanted in a severe combined immunodeficient (SCID) mouse, lacking both functional T and B cells (Sacci J.B. et al. 1992. Proc. Natl, Acad Sci. 89, 3701-3705). This SCID model did not reject the xenograft of human tissue, enabling the transplanted cells to maintain in their host. Subsequent intravenous injections of *P. falciparum* sporozoites led to the infection of transplanted hepatocytes as controlled by immunohistochemical staining at days 1 and 7 after the injection. The first occurrence of liver stage of *P. falciparum* in a mouse transplanted with human hepatocyte was obtained. However, rapidly, these results were found to be disappointing and questionable since two independent research teams had not been able, with the conditions reported in the article, to reproduce the infection, therefore contesting the maturity and functionality of the transplanted hepatocytes (Butcher GA. et al. 1993. Exp. Parasitol 77, 257-260 and Badell E. et al. 1995. Parasitology Today 11 (5), 169-171).

According to another example, in order to evaluate anti-HBV therapeutic agents, a mouse model termed "trimera" was developed (Ilan E. et al. 1999. Hepatology 29(2), 553-562). A normal mouse, preconditioned by lethal total body irradiation and radioprotected with SCID mouse bone marrow, was deemed to be permissive for engraftment of human tissues. The resulting model comprised three genetically disparate sources of tissues. The transplantation of *ex vivo* HBV-infected human liver fragment in such a mouse enabled HBV to replicate for a period of one month, and to generate viremia in the recipient mouse. This model enabled the infected transfected cells to maintain in the recipient and sustained the replication of the pathogen. Such a model also showed the survival of non-infected hepatocytes up to 1 month after transplantation, but not the growth of these latter.

Another strategy was adopted by the team of Ohashi et al. (Ohashi K. et al. 2000. Nat. Med. 6(3), 327-331) to create a xenotransplant model for study of human hepatitis viral infection. NOD/SCID (non obese diabetic/severe combined immunodeficiency) mice were transplanted, in the kidney capsule, with hepatocytes mixed with Matrigel. The loss of the human transplanted hepatocytes was however observed and the hypothesis was made of the absence of an essential growth factor *i.e.*, the hepatocyte growth factor (HGF). The phosphorylation of this growth factor by the addition of a specific antibody against c-met did however stabilize hepatocytes, as shown by measurement of a hepatocyte specific marker concentration *i.e.*, human alpha-1 antitrypsin (hAAT). The authors showed that these hepatocytes had become susceptible to HBV and HDV infection and were able to support the replication of these viruses. However, though this model seemed to be appropriate to study viral infection, only viability and maintenance of transplanted hepatocytes, but no growth, could be observed. Moreover, after about 5 months following transplantation, a 35-40% decrease in hAAT levels was observed, suggesting the persistence of probably less functional hepatocytes.

A mouse model for studying the transplantation of circulating red blood cells (RBC) and their infection by *P. falciparum* was obtained (Badell E. et al. 2000. J. Exp. Med. 192(11), 1653-1659 and Moreno A. et al. 2001. Antimicrob Agents Chemother. 45(6), 1847-1853). Mice bearing mutations affecting T and B cell functions (BXN mice) were treated with intraperitoneal injection of dichloromethylenediphosphonate (Cl₂MDP) encapsulated in liposomes and with anti-polymorphonuclear neutrophils (PMN) antibodies. This treatment enabled survival of *P. falciparum*-infected RBC and enabled the study of drugs in a chronic, stable and long-lasting parasitaemia. This model seemed to be efficient for the survival of without a nucleus and circulating cells, like RBC and nucleated protozoa such as *Plasmodium.*

Another, suitable model for HCV infection, was obtained by Mercer et al. (Mercer D.F. et al. 2001. Nat. Med. 7(8), 927-933). SCID mice (i.e., mice having no functional T and B cells) were crossed with Alb-uPA transgenic mice. These latter express a transgene, the urokinase-type plasminogen activator (uPA) under the control of the albumin promoter, leading to the death of transgene-carrying hepatocytes and resulting in a growth advantage for transplanted cells devoid of said gene. The effectiveness of human hepatocyte transplantation in these crossed mice was controlled by hAAT signal measurement. The results showed that some recipient mice had an extinction of signal around 14 weeks after transplantation, whereas a second subset maintained a strong signal beyond 30 weeks. DNA analysis confirmed that animals with sustained engraftment were homozygous for the transgene, and that the subset with unsuccessful graft was hemizygous for said transgene. This model also demonstrated that murine liver could be repopulated with human hepatocytes, but in the Alb-uPA homozygous mice only. Consequently, the homozygosity of Alb-uPA in this model was deemed to be critical to successful grafting and establishment of viral infection.

Another model showing human hepatocyte partial repopulation of murine liver was that of Dandri et al. (Dandri M. et al. 2001. Hepatology 33(4), 981-988). uPA transgenic mice were crossed with RAG-2 mice (lacking mature T and B lymphocytes), and hemizygous uPA mice were transplanted with primary human hepatocytes. A successful transplantation and partial repopulation (highest degree estimated up to 15% of mouse liver) were obtained with hepatocytes from perfused donor liver specimen. The other experiments with hepatocytes from tissues surrounding tumours or from cell solution failed to produce successful transplantation. Injection of HBV-infectious human serum in uPA/RAG-2 mice resulted in human hepatocyte infection and in presence of viral envelope protein in transplanted mouse serum. Accordingly, the transplanted hepatocytes were permissive for HBV indicating that they are functional. This model proved to be useful in the study of HBV infection when a repopulation could be obtained, *i.e.*, with hepatocytes from healthy livers that underwent a very short ischemia time before perfusion. No transplantation using human hepatocytes obtained from a partial hepatectomy succeeded. This restriction considerably limits the human liver specimens that can be used for transplantation and accordingly the number of efficient models obtained.

The models, presented above, all face important restrictions or drawbacks limiting their use in pathogen and drug studies. Especially, the first above models were easy to produce but only enabled the survival of the implanted human cells and not their growth. The two last models allowing repopulation of hepatocytes were limited by extensive conditions: the requirement for a model harbouring both an immunocompromised trait and a transgene, or the very high quality of implanted hepatocytes.

In order to allow study of pathogens having a specific tropism in human host, models have to fill in conditions that mimic to a large extent those encountered in human. Hence, it would be highly desirable to obtain a model with a degree of repopulation, which would allow cell interactions, and sufficient cell differentiation enabling regular expression of receptors and molecules. This model would be suitable for studying not only pathogen life cycle, but also for the screening of drugs or the design of compounds.

### SUMMARY OF THE INVENTION

In one aspect, the invention provides a method of making a non-human mammal model comprising:
a. implanting, into an immunocompromised non-human mammal host, heterologous nucleated cells previously bound to a biocompatible support,
b. controlling non-adaptive defences of the non-human mammal host,
c. recovering a non-human mammal model harbouring settled heterologous nucleated cells capable of maintaining, differentiating and growing.

Alternatively in such process the above steps of implanting nucleated cells-containing support and controlling non-adaptive defences can be inverted.

The invention also provides non-human mammal model which is an immunocompromised non-human mammal host implanted with a support comprising heterologous nucleated cells settled thereon, and which non-adaptive defences are controlled to enable the heterologous nucleated cells of said implanted support to maintain, differentiate and grow.

Another aspect of this invention provides a tissue matrix comprising set of settled heterologous nucleated cells isolated from a non-human mammal model according to the invention.

In yet another aspect, the invention provides a method for studying a pathogen, in a non-human mammal model of the present invention comprising:
a. infecting said non-human mammal model with a pathogen, in conditions enabling said pathogen to enter in contact with the settled heterologous nucleated cells of the non-human mammal model,
b. observing the pathogen-generated infection in said settled cells.

The invention also relates to the use of the non-human mammal model of the invention for the screening or for the testing of compounds capable of presenting a therapeutic interest.

Another aspect of the invention is a method for screening compounds active against the infection by a pathogen or against its detrimental effects in a non-human mammal model of the invention comprising:
a. infecting said non-human mammal model with a pathogen, in conditions enabling said pathogen to penetrate the settled heterologous nucleated cells of the non-human mammal model,
b. administering the tested compound in conditions allowing its activity to occur,
c. observing the effects of said compound on the pathogen-generated infection or on its detrimental effects.

A further aspect, the invention provides a method for screening the *in vivo* metabolism of xenobiotic compounds, in a non-human mammal model of the invention comprising:
a. administrating the xenobiotic compound to be tested to said non-human mammal model in conditions allowing the compound to interact with settled heterologous nucleated cells,
b. observing its biotransformation at the level of said settled cells.

In a particular embodiment of the invention, the heterologous nucleated cells are human hepatocytes or lymphocytes. Such hepatocytes can be obtained from donor liver specimens, from partial hepatectomy or can be hepatocytes isolated from another non-human mammal model.

A particular immunocompromised non-human mammal host is a SCID, BXN or SCID/Nod mouse.

Particular pathogens for life cycle studies or drug screening is a hepatotropic pathogen such as *Plasmodium* strains *(P.falciparum or P. vivax),* HBV or HCV.

### BRIEF DESCRIPTION OF THE DRAWINGS

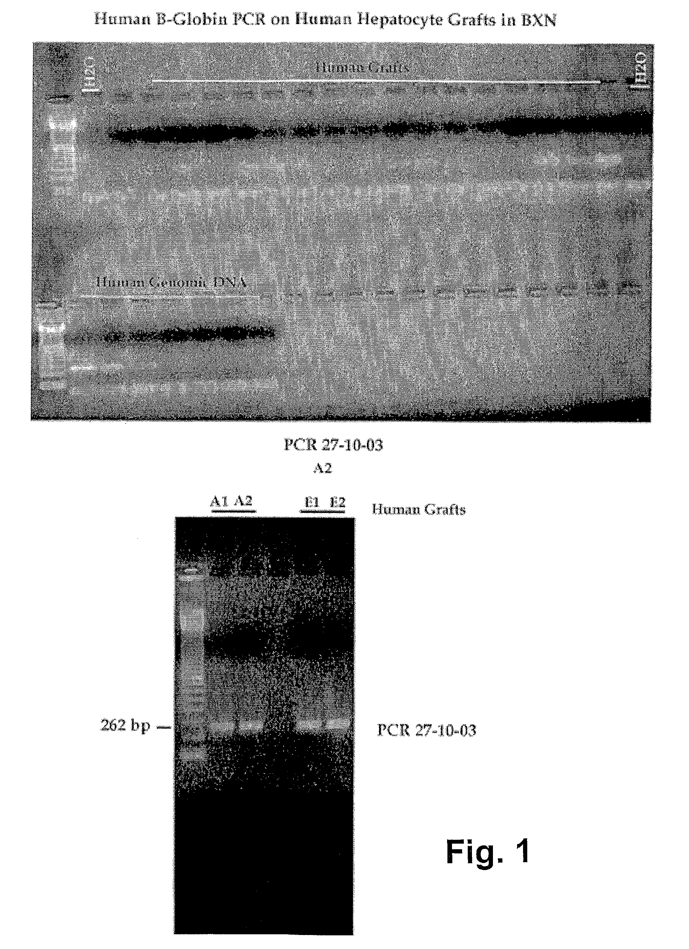

**Fig 1.** Human β-globin PCR on human hepatocyte grafts in BXN mice. All PCR have been done on Human hepatocyte graft in BXN mice.

### a) PCR 27-10-03

PCR using Human β -Globin primers on liver biopsies from grafted BXN (lines 4 to 14) and 2 mice as negative control (lines 15, 16), and on Human liver (Hu Liver, lines 17 to 19) as positive control. PCR without DNA as negative control (line 20, H₂O).

In the same figure is present a "gamme of Human genomic DNA.

### b) PCR 27-10-03

A1, A2, E1 and E2 correspond to Human hepatocytes graft in different BXN mice. PCR using Human β-Globin primers on liver biopsies from grafted BXN (lines 2, 3, 5 and 6)

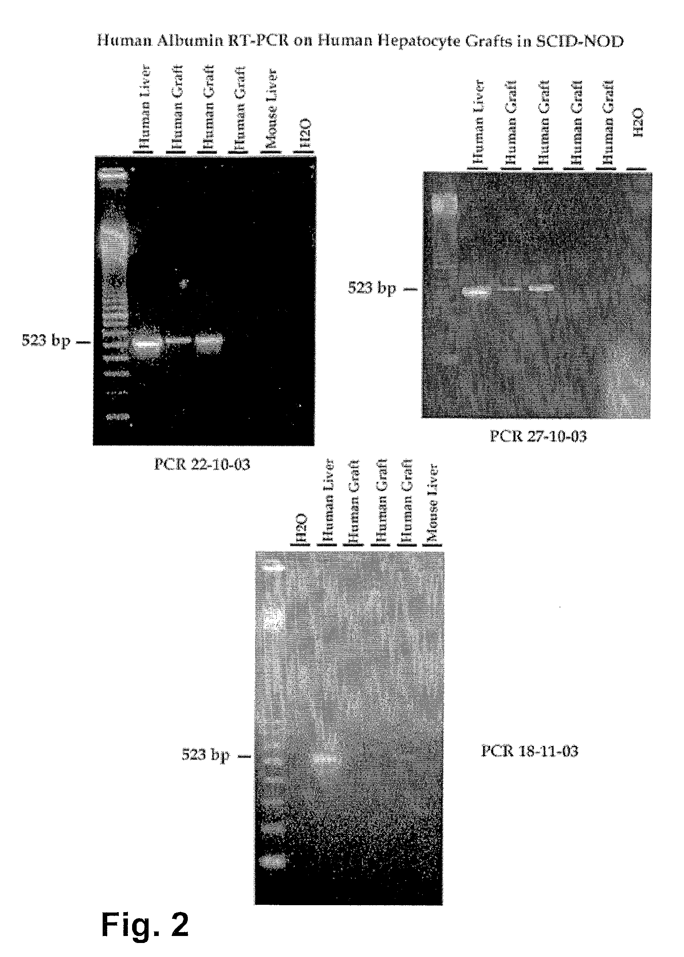

### Fig 2. Human Albumin RT-PCR on human hepatocyte grafts in SCID-NOD mice

All RT-PCR have been done on Human hepatocytes graft in SCID-NOD treated with clorodronate and anti-PMN. At left, appears a 100 bp DNA marker.

### a) RT-PCR 22-10-03

RT-PCR using Human Albumin primers on liver biopsies from grafted SCID-NOD mice (lines 3 to 5) and not transplanted SCID-NOD mouse (line 6) as negative control, and on Human liver (Hu Liver, line 2) as positive control. RT-PCR without DNA as negative control (line 7, H₂O).

### b) RT-PCR 27-10-03

RT-PCR using Human Albumin primers on liver biopsies from grafted SCID-NOD mice (lines 3 to 6), and on Human liver (Hu Liver, line 2) as positive control. RT-PCR without DNA as negative control (line 7, H₂O).

### c) RT-PCR 18-11-03

RT-PCR using Human Albumin primers on liver biopsies from grafted SCID-NOD mice (lines 3 to 5), and on Human liver (Hu Liver, line 3) as positive control. RT-PCR without DNA as negative control (line 2, H₂O).

### Fig 3. Lymphoproliferative response of hu-PBMC against lipopeptides in NIH mice.

Control represents response from cells not stimulated.

PHA represents response from cells stimulated with the PHA mitogen: control for the proliferation of human cells.

Peptide (1, 2 and 3) represents response from cells stimulated by the mixture of peptides.

### Fig. 4. Human Immunoglobulins (hu-lg) detection in NIH mice.

Optical density (OD) calculated for 6 dilutions (1/8 to 1/262144) in six different mice (1 to 6).

### Fig. 5.

Antibody levels (whole lg, lgG and lgM) for 5 different mice for 5 different peptides (NRII, SALSA 1, LSA J, NANP50 and MSP 3). Levels were calculated at different days (Day 6 to Day 88), and for non-treated mice (N).

### DETAILED DESCRIPTION

The present invention provides a method of making a non-human mammal model comprising:
a. implanting, into an immunocompromised non-human mammal host, heterologous nucleated cells-previously bound to a biocompatible support,
b. controlling non-adaptive defences of the non-human mammal host,
c. recovering a non-human mammal model harbouring settled heterologous nucleated cells capable of maintaining, differentiating and growing.

In order to prepare the non-human mammal model starting from said immunocompromised non-human mammal host, heterologous nucleated cells are provided to a support in such conditions enabling said cells to bound on said support and to remain functional. Then, a step is performed to control non-adaptive defences of the host in order to recover in a last step a non-human mammal model enabling the settled heterologous cells to maintain, differentiate and grow.

As an alternative to this method of generating a non-human mammal host, the invention encompasses the possibility that the control of non-adaptive defences is carried out before implanting the nucleated cell bound on said support.

The support considered in the present invention for the implantation step into the mammal non-human host can be of various origins provided said support is biocompatible. Examples of supports are described in EP0702723 patent.

This support is made of a biocompatible material, enabling the biological anchoring of cells (especially by binding, or colonizing the support). As examples of support one may cite synthetic biocompatible materials such as polytetrafluoroethylene fibers (PTFE), materials of biological origin such as calcium carbonate and preferably coral or such as cross-linked collagen fibers.

The heterologous nucleated cells can be attached to the surface of the support or can penetrate into the interior of this support to achieve settlement.

The binding of the cells to the support is allowed in particular by the presence of constituents capable of inducing and/or promoting the inclusion of the cells within a matrix having the constitution of a gel (by a process called gelation). Such gel comprises collagen gel, for instance rat-tail collagen, bovine collagen or human collagen.

The materials to build convenient supports may or may not be resorbable by the host into which they are introduced.

The mammal considered in this invention either as host or as model can be any animal of the mammal group, except human, provided it is relevant for use in the context of the invention.

"Model" as used herein relates to a non-human mammal host, which comprises nucleated cells from a xenogenic origin i.e., originating from a different organism, in particular originating from a different animal species. In a particular embodiment of the invention, said nucleated cells are human cells. When implanted in the host, the cells maintain, differentiate and grow.

"Host" as used herein is a non-human mammal and immunocompromised because of altered immunologic mechanisms resulting from genetic mutations, treatments or surgery.

"Implanting" as used herein is the process of incorporating the support containing heterologous nucleated cells into a recipient non-human mammal host. The implantation can take place in various locations, e.g., intrahepatic, intrasplenic, intraperitoneal or intraorbital and the implanted cells when settled in the non-human mammal model can circulate or to the contrary can remain at a determined location. Implantation can be persistent or transitory.

Before settling the support, cells are treated according to techniques well known to those of skill in the art. Cells can be primary cells directly deriving from an organ resection, cells having previously undergone various treatments, including genetic modifications, cells from another non-human mammal model or from an *in vitro* culture.

"Nucleated cells" as used herein are cells that contain a nucleus. In a particular embodiment, cells are used that are capable of having a differentiation activity and/or of dividing to repopulate in the host. Such cells can be hepatocytes, lymphocytes.... In a particular embodiment, the cells used are stem cells or pluripotent cells.

The model can be used to implant, several nucleated cell types, and also, besides nucleated cells, enucleated cells such as red blood cells (RBC).

The implanted cells are healthy cells and accordingly encompass non-infected and non-tumoral cells. The implanted cells also encompass mutated or recombinant cells.

"Non-infected" cells refers to cells that have not undergone, previously to the implantation, interactions with the pathogen which effect on said cells may be tested later in the obtained model. Such cells are for example obtained from an organ of a patient who has been tested negative for said pathogen or whose background enables to support that he was free from infection for the period of concern (*e.g.* histological and/or biological signs).

For example when the invention provides a non-human mammal model, which comprises human hepatocytes, the implanted cells are not infected by *Plasmodium, e.g., Plasmodium falciparum,* and/or by HVB and/or by HCV.

"Non-tumoral" cells refer to cells having controlled cellular proliferation and spread and having a stable karyotype, *i.e.* cells containing the same number of chromosomes after multiple divisions.

In the invention, the herologous nucleated cells, although they are non-infected, and non-tumoral cells can nevertheless carry a mutation which effect may be studied when these cells are brought in contact with a determined compound administered to the model. They can also be recombinant cells as a result of incorporation of a heterologous sequence which impact on the cells is to be tested.

"Non adaptive defences" refer to cells involved in the non-specific immunity, such as macrophages, monocytes or polymorphonuclear neutrophils (PMN), in contrast to specific immunity directed by T and B lymphocytes.

"Settled" as used herein refers to cells that are not lost after the implanting step of the support comprising the same, and refers accordingly to cells that succeed in surviving in and repopulating the non-human mammal model.

The capacity of implanted cells "to maintain" as used herein refers to the capacity for the implanted cells to survive in the host.

The capacity of implanted cells "to differentiate" as used herein refers to cells having the capacity to reach, after the implanting step, characteristics as similar as possible to those of the same cell type in their original host. This capacity can be determined in terms of secreted molecules (such as albumin for the hepatocytes), expressed surface receptors, pathogen infection, cell size or any other appropriate methods. The presence of human cell type-specific molecules expressed by the settled cells can be measured, on sera, by well known techniques such as ELISA (enzyme-linked immunoabsorbent assay), Western Blot, dot blot, immunoprecipitation, direct or indirect immunostaining on histological sections using specific antibodies of implanted cell markers The cell type-specific molecule transcripts can be detected by RT-PCR (reverse transcriptase-polymerase chain reaction) or real-time RT-PCR by using specific primers of implanted cell markers. Specific receptors can be detected by various techniques such as FACS analysis. Finally, pathogen infection is controlled by detection of settled cell stage specific proteins by techniques including light microscopy, immunohistological staining on biopsies, by ELISA, PCR or RT-PCR on sera and by Western Blot, PCR or RT-PCR on cellular extracts.

The capacity of implanted cells "to grow" as used herein refers to the capacity of settled cells, not only to survive in the recipient but also to multiply in the obtained model. Growth can be measured by quantitative imaging and evaluating the percentage of cells expressing a specific cell type marker. Measurements can be made at different time points to follow the repopulation of the settled cells. The growth can also be followed by the analysis of the DNA synthesis by the incorporation of a labelled nucleotide such as BrdU.

One advantage of the invention lies in the fact that the only required characteristic of the non-human mammal host used to prepare the model is the immunocompromised trait. There is no need for a host bearing several genetic defects, and therefore reduce the number of crosses necessary between different strains to obtain a host able to be implanted. Consequently, this leads to a faster and cheaper generation of the required host.

The inventors have determined that controlling non-adaptive defences of the host is one of the parameters enabling the implanting cells to settle, differentiate and grow in the non-human mammal model.

The efficiency of the control of non-adaptive defences can be checked by various techniques, such as FACS analysis. Essential actors involved in the non-adaptive defences are macrophages and PMN for which a strong reduction or depletion is expected after immunomodulation treatment according to the invention.

"Macrophage depletion" as used herein is the process of reducing in a large amount but not totally the circulating and tissue macrophages. A convenient range of remaining macrophages after treatment is 0% to 50%. A particular range of remaining macrophages is 0% to 20%.

Macrophage number can be reduced by administrating, in the host, antagonists of macrophages, such as toxic substances, like cis-platinium, or antibodies, altering macrophage development or function and finally killing them. The administration of antagonists is performed by well-known techniques, including the use of liposomes. The reduction of macrophages can also be reached by irradiation.

"PMN depletion" as used herein is the process of reducing polymorphonuclear neutrophils (PMN) cells after treatment. A convenient range of remaining PMN after treatment is 0% to 50%. A particular range of remaining PMN is 0% to 20%.

The administration of PMN antagonists or substances altering their function and development is made by well-known techniques including by using vectors including liposomes.

In a particular embodiment, the macrophage depletion is obtained by injecting liposomes containing Cl₂MDP according to the technique of Van Rooijen et al. (Van Rooijen N. 1989. J. Immunol. Methods 124, 1-6). The liposome size can range from 0.5 to 7 µm to be ingested by macrophages, resulting in their killing.

The PMN depletion is preferably performed by injecting an anti-PMN antibody, such as the NIMP-R14 monoclonal antibody, which not only depletes part of the PMN but also blocks the function of the remaining so that PMN activity is in total strongly reduced or abrogated. The activity of the NIMP monoclonal antibody induces, in particular, a disappearance of cytoplasmic neutrophil granulations that are normally released by PMN when they are in contact with a pathogen.

A particular protocol of macrophage depletion is the injection of Cl₂MDP embedded in liposomes, at 4-day interval, starting two days after implanting. Such liposomes fully clear macrophages from peritoneum, liver, spleen and kidney. Monocytes from the bone marrow colonise the liver after the clearance of all Kupfer cells and transform into very active large macrophages and new Kupfer cells. These cells are, again, destroyed by the next injection of liposomes.

In a particular embodiment for PMN depletion, anti-PMN antibodies are injected at monthly interval, starting two days after implanting. The interval between injections is 3 to 4 days.

In a particular embodiment, the non-human mammal host used for the generation of the model is a rodent and particularly mice, especially because of their low price, the easiness in breeding and the various strains available.

A particular immunocompromised host for the implanting step is the SCID mouse (severe combined immunodeficiency), the SCID/Nod mouse (severe combined immunodeficiency/non obese diabetis) or mammals with altered lymphocyte lineages such as the BXN (NIHIII or Beige Xid Nude), the RAG, the RAG2 and the RAG-γC mouse.

In a particular embodiment, mice are implanted with a support comprising human hepatocytes. The cells can be prepared as described (Dandri M. et al. 2001. Hepatology 33, 981-988) by collagenase treatment. However, they can be kept under *in vitro* conditions in cultures (*e.g.*, one to 3 weeks) before the implanting step into the animals. In a particular embodiment, the binding and implanting steps are performed with adult or foetal primary hepatocytes, bone marrow cells or hepatocyte cell lines.

The present invention provides also non-human mammal model which is an immunocompromised non-human mammal host implanted with a support comprising heterologous nucleated cells settled thereon, and which non-adaptive defences are controlled to enable the heterologous nucleated cells of said implanted support to maintain, differentiate and grow.

An advantage of the non-human mammal model is not only the maintenance of the settled nucleated cells, but also their growth and differentiation.

The growth and differentiation of the settled nucleated cells of the model of the invention can be checked by various features, such as the presence of cell surface receptors, the secretion of proteins specific of the implanted cells, the cell size or the receptivity to pathogens.

In a particular embodiment of the invention, the model enables the settled nucleated cells to secrete specific proteins characterizing the differentiation and growth of the settled cells, for several months. Specific proteins of the implanted cell type such as albumin for hepatocytes, ... can be used as markers to follow the differentiation state as well as the repopulation.

The hypothesis that implanted cells acquired a differentiation state similar to that observed in *in vivo* conditions, can be tested by the stringent requirements of some pathogens to infect differentiated cells to permit their development.

Another advantage of the model of the invention is the receptivity of settled cells for pathogens having a restricted tropism.

"Receptive" as used herein refers to the capacity of settled cells to sustain an infection similar to that observed in their original host. Therefore, pathogens penetrate settled cells and realize their replication and maturation.

The non-human mammal model of the invention comprising settled heterologous nucleated cells is suitable for use in studying pathogens with specific tropism for these cells.

According to another embodiment of the invention the model is suitable for *in vivo* study of metabolism of administered compounds and especially drugs, in said settled cells.

As a particular embodiment, the mammal host is a mouse, implanted with human hepatocytes. In this mouse model, settled cells are receptive to hepatotropic pathogens, such as HBV, HCV or *Plasmodium* strains, *e.g. P. falciparum* or *P. vivax.*

In another particular embodiment, the mammal host is the mouse implanted with lymphocytes. In this model, the injection of peptides derived from pathogens leads to the production by the implanted lymphocytes of antibodies specific of these peptides.

The invention provides also a tissue matrix generated from the non-human mammal model. "Tissue matrix" as used herein refers to a set of settled heterologous nucleated cells coming from a non-human mammal model according to the invention. This settled cell set can appear:
- as isolated cells in suspension and treated by well known techniques, such as centrifugation on Percoll gradient,
- as "solid" preparations such as biopsy fragments
- as cell line obtained after adhesion of the settled cells on a substrate. The substrate can be of synthetic origins, including biodegradable or biostable polymers, natural origin or a mixture of both, and is chosen to maintain the normal biological activity of the cells. Examples of such and artificial substrate are plastic, glass or membrane. Cells can also be cultured on reticulated components such as collagen, gel or reticulated polymers.

This tissue matrix forms a constant and homogeneous reserve of differentiated non-human mammal cells. An advantage of the tissue matrix is that cells, constituting it, can be used for a new implantation. Consequently, it is not necessary to obtain new tissues or new biopsies.

The non-human mammal model of the present invention allows the study of restricted-tropism pathogens, for which no permissive line exists or for which permissive models are not fully satisfactory. The non-human mammal model of the present invention contains settled cells that are differentiated and are able to sustain a pathogen infection for several weeks. Because of the long duration of the settled cells survival, their repopulation and their high number in the non-human mammal model, pathogen life cycle can be intensively studied including when said infected cells are put in contact with candidate drug compounds.

The invention also provides a method for studying a pathogen, in a non-human mammal model of the invention comprising:
a. infecting said non-human mammal model with a pathogen, in conditions enabling said pathogen to enter in contact with the settled heterologous nucleated cells of the non-human mammal model
b. observing the pathogen-generated infection in said settled cells.

The first step is the introduction of a pathogen in the non-human mammal model, in conditions in which it can interact with settled cells and especially can penetrate in them. The introduction of the pathogen can be achieved by various ways, including intravenous or intracutaneous injections.

In a second step, the infection of said settled cells is monitored by well known methods including, but not limited to, light microscopy, immunofluorescence antibody test (IFAT) using pathogen specific antibodies, PCR (qualitative or quantitative) or RT-PCR using primers for a pathogen specific gene, ELISA or immunoprecipitation. When a pathogen has a life cycle with different forms infecting various organs or various species, antibodies and primers are chosen to be specific to proteins of each form, and in the invention, specific of the proteins of the settled cell form. All the results obtained to identify the pathogen infection, for example the number of pathogens calculated by light microscopy, the staining observed by IFAT and the mRNA transcripts detected in the settled cells of the model, are compared to a control model infected by the same pathogen but without cell implantation.

In a particular embodiment, the non-human mammal tested is a mouse model generated from a SCID mouse host in which a human hepatocytes-containing support is implanted.

Particular pathogens are hepatotropic pathogen including HBV (hepatitis B virus), HCV (hepatitis C virus) or *Plasmodium* strains including *P. falciparum* and *P vivax.*

The monitoring of the hepatocyte infection by *P. falciparum* can be performed by testing pathogen proteins, specific for hepatocytes and/or erythrocytes, such as the circumsporozoite protein (sporozoite), the MSP3 protein (erythrocytes), the HSP70 and MSP1 proteins (hepatocytes and erythrocytes) and the LSA1 protein (hepatocytes).

The monitoring of the hepatocyte infection by HCV can be performed by measuring the presence or absence of the viral RNA sequence (qualitative PCR) or the viral load (quantitative PCR).

The monitoring of the hepatocyte infection by HBV can be performed by quantifying a viral envelop protein HbsAg with the ELISA technique.

The invention provides also the use of a non-human mammal model of the invention for the testing of compounds presenting a therapeutic interest towards the infection or its consequences.

The non-human mammal model can also be useful for the screening of drugs capable of altering the life cycle of pathogens. Owing to the capacity of the non-human mammal model to sustain a pathogen infection for several weeks, the effects of a drug administration on the infection can be observed and its efficacy can be evaluated. The invention provides a method for screening active compounds against the infection by a pathogen or its detrimental effects in a non-human mammal model of the invention comprising:
a. infecting said non-human mammal model with a pathogen, in conditions enabling said pathogen to penetrate the settled heterologous nucleated cells of the non-human mammal model,
b. administering the tested compound in conditions allowing its activity to occur,
c. observing the effects of said compound on the pathogen-generated infection or on its detrimental effects.

In a first step, settled cells are infected according to the method described above.

Then, the drug is administered to the non-human mammal model in conditions in which the drug keeps, modifies or acquires its activity. The interactions with the settled cells as well as the host environment could be determined with respect to the activity of the drug.

The drug can be administered under any appropriate forms including systemic or local routes.

Several drugs (at least two) can be administrated together, alternatively or with specific protocols to show a possible synergy, redundancy or antagonism.

The drug can be administrated by a lot of well known routes, including taken by mouth (orally), given by injection into a vein (intravenously), into a muscle (intramuscularly), beneath the skin (subcutaneously) or placed under the tongue (sublingually), inserted in the rectum (rectally) or vagina (vaginally), instilled in the eye (by the ocular route); sprayed into the nose and absorbed through the nasal membranes (nasally); breathed into the lungs (by inhalation) or applied to the skin (cutaneously).

Finally, the effects of the drug on the pathogen infection or on its detrimental effects can be monitored. Various techniques can be used to observe the effects of the administrated drug, including light microscopy, immunofluorescence antibody test (IFAT), RT-PCR, PCR (qualitative or quantitative), or ELISA. The infection are followed at different time points and various factors can be calculated: drug half-life, minimal effective dosage for infection elimination or reduction, drug dosage based on age, on body weight or on body surface area, the most efficient place or route for drug administration, the combination therapy efficiency. Other factors that can be observed could be the biological activity of the drug metabolites produced by settled heterologous cells as well as the toxicity on the various organs of the non-human mammal of these specific metabolites.

The invention also provides a method for screening the *in vivo* metabolism of xenobiotic compounds, in a non-human mammal model of the invention comprising:
a. administrating the xenobiotic compound to be tested to said non-human mammal model in conditions allowing the compound to interact with settled heterologous nucleated cells,
b. observing its biotransformation at the level of said settled cells.

This subset of heterologous nucleated cells can be exploited to follow the biotransformation of a xenobiotic, after its injection into the non-human mammal model. "Xenobiotic" as used herein refers to a chemical substance (or more generally, a chemical mix) that is not a normal component of the organism in which it is exposed to. Xenobiotics include most drugs (others than those compounds which naturally occur in the organism), as well as other foreign substances.

The xenobiotic is administrated into the non-human mammal model according to all routes and all forms cited above for the drug. One condition in the administration is that the xenobiotic can interact with the settled cells.

The measurement of the level of the metabolites (degradation products), including intermediates and final products, enables to track the xenobiotic metabolism kinetics, including its half-life. The model enables also to observe the effects of the compound on the settled cells, to evaluate the doses at which the effects appear. Finally, it is possible to study the potential interactions between reactive metabolites and cellular macromolecules.

As most xenobiotic compounds are metabolised by the liver, a particular mammal tested is a mouse model generated from a SCID mouse host, in which a hepatocyte-containing support is implanted. In this model, one could monitor the cytotoxic effects of the drug on the liver, e.g., by measuring the circulating hepatic transaminases and by analysing the liver histology with optical microscopy techniques.

The invention also provides technical platforms comprising at least the chimeric murine model of the invention such as:
- a technical platform, useful to identify new compounds useful to treat mammal infections provoked by a pathogen, characterized in that it comprises at least a chimeric model as defined above and appropriate means to detect or to observe the effects of said compounds on a pathogen-generated infection of said model.
- a technical platform, useful for screening the *in vivo* metabolism of xenobiotic compounds characterized in that it comprises at least a chimeric model according to the invention and appropriate means to observe the biotransformation of said compounds by implanted human cells in said murine model.

### EXAMPLES

Examples are given to illustrate but not to limit the invention.

### Example 1: generation of a mouse model grafted with hepatocytes

### Animals

6-8 weeks male and female BXN, SCID and SCID/NOD mice, purchased from IFFA-CREDO, were kept in sterile isolators and provided with autoclaved tap water and a γ-irradiated pelleted diet ad libitum. Mice were housed, maintained and manipulated under pathogen-free conditions in laminar-flux hoods. All animals were treated according to laboratory animal guidelines.

### Isolation of human hepatocytes

Primary human hepatocytes were isolated as described elsewhere (Guguen-Guillouzo C. et al. 1986. Prog Liver Dis 8, 33-50) from the healthy liver tissue of surgical liver biopsies specimens (approx. 20-25 cm³) obtained with informed consent from patients who underwent therapeutic partial hepatectomy for liver metastasis and benign hepatic tumor, according to French National ethical regulations (article L-1245-2 of the Huriet laws). Subjects with viral infections (HCV, HBV, HIV), cirrhosis and primary hepatic carcinoma were excluded.

Resected liver lobes were cut at a distance of at least 3 cm from the metastasis. Human hepatocytes were isolated by a two-step perfusion technique. Hepatocyte viability obtained by this method depends on 3 important factors which are the temperature: 37°C, pH: 7.6 and perfusate flow rate: 16-20ml according to the rejection size. Perfusion began with a HEPES buffer, without calcium, at a temperature, a pH and a perfusate flow rate as indicated above. Hepatocytes were then isolated with a perfusion of 200 ml of HEPES buffer supplemented in Collagenase H 0.05% (Roche Molecular Biochemicals) and CaCl₂ 5 mM, and separated from non-parenchymatous cells by Percoll fractionation, as previously described (Giannini C. et al. 2003. Hepatology 38, 114-122; Guguen-Guillouzo C. et al. 1993. Cytotechnology 11 Suppl, S3-5; Guguen-Guillouzo C. et al. 1982. Cell Biol Int Rep 6(6), 625-8). Viable cells were determined by trypan blue exclusion.

### Implantation of human hepatocytes

Mice were anesthetized by intra-peritoneal injection of 0.3 mg Valium (10mg/kg), followed by 2.5 mg Ketamin (83.3 mg/kg) for surgical implantation of collagenase-dissociated human hepatocytes dispersed in a collagen matrix mostly in peritoneal cavity.

After dissociation of hepatocytes with the collagenase, 1 to 10 millions of cells were injected in a collagen matrix (sponge-like), which was then sutured to the epiplon and to the peritoneal cavity to facilitate the vascularisation of the "neo-organ" or to the epiplon plus the intestine to facilitate bile ducts formation and bile evacuation. All surgery and animal handling procedures were done using a strict aseptic technique in a laminar flow hood.

### Immunomodulation Protocol

The implantation of human hepatocytes in mice host induces a strong increase in tissue macrophages, particularly in the liver, the spleen, and the peritoneal cavity, as well as circulating polymorphonuclear neutrophils (PMN) and monocytes.

Dichloromethylene diphosphonate (Cl₂MDP) encapsulated in liposomes were used as described previously (Nico Van Roojen 1989. J.Immunol.Methods 124, 1-6). The liposome-mediated macrophage "suicide" technique, allowed the reduction of tissue macrophages in BXN, SCID-NOD mice in response to the presence of heterologous cells.

The increase in PMN was controlled by using a NIMP-R14 monoclonal antibody (Lopez A. et al. 1984 Br J Haematol 57(3) 484-94). Mice were injected intraperitoneally at 4-day interval starting 2 days after transplantation with liposome-encapsulated clorodronate (100 µl of a solution at 50% hematocrit of liposomes) and antibodies NIMP-R14 (100 or 200 µg/ml) every 3 to 4 days. Clorodronate was commercialized by Roche Diagnostics GmbH and encapsulated as described earlier.

### Example 2: Human liver cells/Hepatocyte detection

15 days to 9 months after transplantation, mice were sacrified. Liver graft was removed and processed for histology and/or human DNA detection by PCR. Human albumin detection was assessed by RT-PCR and by ELISA performed on mice sera.

### Detection of human DNA within the graft

Genomic DNA was isolated using the GenElute Mammalian Genomic DNA (Kit, from Sigma), and Human β-Globin amplified by PCR using β-Globin specific primers: 5'-GGTTGGCCAATCTACTCCCAGG-3' (KM29) and 5'-TGGTCTCCTTAAACCTGTCTTG-3' (KM38).

Human peripheral blood served as a positive control and non-transplanted BXN liver served as negative control. PCR conditions were 95°C for 5 min; 94°C for 30s, 55°C for 30 sec, and 72°C for 30 sec for 40 cycles, with a final extension at 72° C for 5 min. Twenty microliters of final PCR product were analyzed by electrophoresis (2% agarose gel with Ethidium Bromide) and PCR product bands (262 bp) were visualized under UV trans-illumination.

### Detection of Human albumin RNA within the graft

For human albumin detection, total RNA was isolated from human-mouse chimeric hepatocytes, murine and human liver cells tissues, using RNeasy Protect Mini kit (Qiagen) according to the manufacturer instructions. Purified RNAs were quantified spectrophotometrically, and equal amounts of RNA from each sample were subjected to cDNA synthesis using random primers. The following human specific albumin primers, 5'-CATTAGCTGCTGATTTTGTTGAAAG-3' and 5'-TGTGCAGCATTTTGTGACTCTG-3' were used to detect human albumin transcripts. PCR Conditions were 95°C for 5 min; 94°C for 30s, 60°C for 1 min, and 72°C for 1 min for 40 cycles, with a final extension at 72° C for 5 min. Twenty microliters of final PCR product were analyzed by electrophoresis (2% agarose gel with Ethidium Bromide) and PCR product bands (523 bp) were visualized under UV trans-illumination.

### lmmunohistochemistry

Human liver grafts fixed in Zn buffer were paraffin embedded. Sections (5 µm) were Hemotoxylin-Eosin stained in standard fashion. To detect human hepatocytes and other cells specific to the liver, sections were immunostained with a monoclonal antibody against human albumin (Clone 3H5/G4, or anti-human albumin, Sigma), Human Kupffer cells (CD68, Dako), Human Endothelial cells (CD31, Dako), Human Biliary duct cells (CK19, Dako), with bound antibody detected by an anti-mouse IgG-(H+L) Alexa 488 (Molecular probes).

### Detection of human albumin in grafted mouse serum by ELISA (Enzyme-linked immunosorbent assay)

Human albumin was detected *in situ,* on cultured human hepatocytes recovered from the graft and/or in the serum of grafted mice (reflecting the differentiation status of the grafted hepatocytes) by the use of a monoclonal anti-human serum albumin clone HAS-9 (Sigma, St Louis, USA) at a serum dilution of 1/10.

Following overnight coating at 4°C of 100 µl of anti-human serum albumin (clone HSA-9) diluted at 1/100, non-specific binding was blocked by incubation with 1% bovine serum albumin for 1 hour at 37°C. After washing, 75 µl of HRP-conjugated rabbit anti-human albumin diluted at 1/8000 (0.16 µg/ml, Sigma Chemical Co.) was incubated overnight at 4°C. HRP-conjugated rabbit anti-human albumin was used as antigen-specific indicator antibodies. The chromogen and the substrate were used according to the manufacturer indications (Sigma Chemical Co.) (OPD tablet in 1X Citrate Buffer). Absorbance values (405 nm) were converted to concentrations in µg/ml by comparison with a standard curve performed by using serial dilutions of defined amounts of purified human albumin (Sigma Chemical Co.). The ELISA result was considered as positive for the detection of human proteins (HA, hα1AT) when the OD value was higher than the OD means + 2 fold the standard deviation of 14 non-implanted Alb-uPA/SCID mice. Qualitative comparisons were done using the chi 2 test and Fischer exact test. P values of less than 0.05 were considered as significant.

### Example 3: Results obtained in BXN mouse model(1)

A group of 11 BXN mice, without complementary immunomodulation treatment, were grafted with dissociated, isolated hepatocytes within an extra-cellular matrix made of collagen sponges in intra-peritoneal location.

Examination of the biopsies, 1 month and half after grafting, showed that the neo-organ was vascularised, and had increased in size up to 2 times (from about 3 mm to 7-8 mm in diameter).

Hepatocyte survival was obtained and was ascertained by a perfusion by collagenase of the neo-organ, cultivation of the hepatocytes and detection of human albumin.

These results supported the idea that the long-term survival of human hepatocytes was achievable in immunodeficient mice.

In these mice, that did not receive anti-PMN antibody treatment, the presence, of a large ring made mostly of polymorphonuclear cells, particularly visible in biopsies grafted in the muscles, suggests both that these cells are critical in defence in mice lacking B and T lymphocytes and, given the duration of survival of the hepatocytes, that hepatocytes were most likely replicating at the same time as PMN were destroying the peripheral ones.

### Example 4: Results obtained in BXN mouse model (2)

The next group was made of 16 BXN mice without complementary immunomodulation treatment, receiving isolated hepatocytes in extra-cellular matrix in the peritoneal cavity. They showed morphological features of hepatic cells.

However, labelling by a large variety of markers led to non-conclusive results with high background which was later on explained by suffering of the cells in the absence of immuno-complementary treatment leading to a non-specific binding of the antibody.

Human β-Globin PCR, performed on extract from these 16 BXN mice grafted with human hepatocyte revealed that 11 out of 16 grafts were found positive for human β-Globin DNA from 15 days to 9 months after transplantation (Figure 1).

The presence of hepatic tissue at 9 months indicates that the implanted hepatocytes have an active multiplication, despite the non-adaptative defences in those mice. It also stressed the value of the complementary treatment aimed at depleting the activity of tissue and circulating macrophages.

### Example 5: Results obtained in SCID-NOD mouse model

10 SCID-NOD mice were grafted with dissociated hepatocytes in extra-cellular matrix in the peritoneum. They all received complementary treatment by anti-PMN antibodies and CL₂MDP containing liposomes, 2 days post-grafting.

The comparison between mice receiving anti-PMN and macrophages depletion treatment and those receiving no complementary treatment showed a major improvement in the size of the recovered graft one month after grafting as well as in the morphology of the recovered hepatocytes in hematoxylin-eosin coloured sections.

They also show a drastic decrease in the number of PMN infiltrating or surrounding the grafts. The absence of non-specific labelling by immuno-histochemistry in those grafts is another proof of the better health of the recovered hepatocytes.

2 biopsies have been analysed by PCR for the presence of human genomic DNA (using primers corresponding to a γ-globin gene), 2 out of 2 were positive.

Human Albumin RT-PCR was performed on extract from 10 human hepatocyte grafts of the treated SCID-NOD mice. Human albumin messenger was detected in 6 out of 10 grafted mice from 15 days to 4 months post-transplantation (Figure 2).

### Example 6: generation of a mouse model grafted with lymphocytes Isolation and implantation of human lymphocytes

Human lymphocytes were obtained from blood of human umbilical cord. Total humans peripheral blood mononuclear cells (hu-PBMC) from healthy donors were isolated by a gradient of ficoll-hypaque (Jacques Boy, France). Cells were washed twice with Hank's solution buffered with Hepes (Gibco, BRL) and 3x10⁷ cells/mice were grafted in the peritoneal cavity of BXN-NIH III mice (Charles River)

### Immunomodulation Protocol

Dichloromethylene diphosphonate (Cl₂MDP) encapsulated in liposomes were used as described previously (Nico Van Roojen 1989. J.Immunol.Methods 124, 1-6). The liposome-mediated macrophage "suicide" technique, allowed the reduction of tissue macrophages in BXN, SCID-NOD mice in response to the presence of heterologous cells.

The increase in PMN was controlled by using a NIMP-R14 monoclonal antibody (Lopez A. et al. 1984 Br J Haematol 57(3) 484-94). Mice were injected intraperitoneally at 4-day interval starting 2 days after transplantation with liposome-encapsulated clorodronate (100 µl of a solution at 50% hematocrit of liposomes) and antibodies NIMP-R14 (100 or 200 µg/ml) every 3 to 4 days. Clorodronate was commercialized by Roche Diagnostics GmbH and encapsulated as described earlier.

### Immunisation of mice.

We used a cocktail of peptides constituted by three lipopeptides from proteins of pre-erythrocytic stages of *Plasmodium falciparum:* NRII (from LSA-3 antigen), LSA-J (from LSA-1 antigen) and SALSA-1 (from SALSA antigen). They where chosen because of their excellent immunogenicity in Chimpanzees, Aotus monkeys and mice. We intraperitoneally (i.p.) injected 50 µg/mouse of each lipopeptide per immunisation without adjuvant.

### Protocols of immunisation.

Different forms of immunisation were tested. Two mice received, at day 0, the hu-PBMC incubated during 15 minutes with the pool of lipopeptides before its i.p injection (mice N°1 and 2). Two other mice (mice N°3 and 4) received the i.p injection of the pool of lipopeptides a day after the hu-PBMC injection. Two more mice (mice N°5 and 6) also received the injection of lipopeptides at this time but the cells were placed in a sponge of collagen, which was then introduced into the peritoneum by a surgical operation under Valium/Ketamine anaesthesia. Further two immunisations were performed i.p. in all mice at days 10 and 20.

### Example 7: lymphocyte detection

Lymphocytes are then collected in the peripheric circulation of the grafted mice and detected according to the following techniques:

### Flow cytometry analyse:

Circulating human CD2⁺ CD3⁺ cells were detected in the mouse blood by FACS using a monoclonal antibodies anti-CD2 anti-CD3 coupled to fluorescein (DAKO A/S, Denmark.). A monoclonal antibody of the same isotype was used as a control. Blood was collected on heparin from the retro-orbital sinus of mice. hu-PBMCs were isolated by ficoll-hypaque (Jacques Boy, France) gradient. After washing, cells were incubated 30 min with the monoclonal antibody containing 1% of mice serum. After washing (NaCl 0,9%-hepes, 400 x g, 10'), cells were suspended in 0.3 ml NaCl 0,9%-hepes. The % of positives cells was detected by cytometry (EPICS MCL-XL, COULTER-COULTRONICS).

### Lymphoproliferative assays

50 µl of hu-PBMC and 50 µl peptides per well were plated into 96 conic wells plates. Antigens were pooled at 10 µg/ml each and cells were at 2 x 10⁴ cells/well. As mitogens, we used PHA at 2 µg/ml. All dilutions were made in RPMI-1640 (GIBCO, BRL, France) supplemented with penicillin/streptomycin, non-essential amino acids, sodium piruvate, hepes and 10% of human AB serum. All tests were performed in triplicate. Plates were incubated in a humid incubator at 37 °C and 9% of CO2. 1 µCi of 3H-thymidin per well was added 6 days later. Incubation was continued by 12-18 hours and them the cells were harvested on a fiber glass sheet using a cell harvester (Tomtec, ECG Instruments) and counted in an scintillation counter (Microbeta, ECG Instruments).

### Detection of human immunoglobulins (hu-lg)

The detection of hu-lg was performed by the ELISA technique. Briefly, plates were coated with 50 µl/well of antigen solution incubated at 4°C overnight. Then, the plates were washed and incubated 2 hours at room temperature with a solution containing milk without fat (300 µl/well). After 3 washes, plasma from mice were plated and incubated 1 hour at 37°C. Plates were washed 5 times and anti-human antibodies against whole hu-lg, anti-γ and anti-µ, coupled to alkaline phosphatase (Immunotech, Marseille, France) were added and incubated 1 hour at 37°C. After 5 washes, 50 µl/well of p-nitrophenyl phosphate (Sigma, St. Louis) 1mg/ml in glycine buffer were added and incubated 15-30 min in a dark place. Finally, the optical density (OD) was read at 405 nm.

### Peritoneal and spleen cell isolation

The mice were killed by cervical dislocation. Peritoneal mononuclear cells were obtained injecting 10 ml of cold NaCl 0,9%-hepes into the peritoneal cavity. After massing of the mouse abdomen, the suspension of cells was aspirated and the mononuclear cells were isolated by ficoll-hypaque gradient (Jack Boy) as described above. The isolated cells were centrifuged at 225 x g for 10 min at 5 °C, resuspended in RPMI 1640 medium with 5% fetal calf serum, with penicillin 100 U/ml and streptomycin 100 µg/ml (RPMI/FCS/P-S) (GIBCO BRL, Life Technologies). Then cells were counted by visual hemocytometer and aliquoted for flow cytometry staining.

Spleen cell suspensions were prepared by pressing the spleen between the frosted ends of glass microscope slides to disrupt the tissue by gentle shearing pressure, and were rinsed into RPMI/FCS. A pool of spleen cells from two mice was mixed, and the large debris were allowed to settle for 5 min at 5 °C. Then, the supernatant cell suspension was removed and centrifuged at 225 x *g* for 10 min at 5 °C. The cell pellet was resuspended in 1 to 2 ml of RPMI/FCS, and counted by hemocytometer.

### Example 8: Results obtained in mouse model

### In vitro proliferative response of mononuclear cells against lipopeptides

We tested the *in vitro* response of PBMC isolated from peripheral blood of humanised mice. This test was performed with a mixture of three peptides because of the low number of cells obtained after separation.

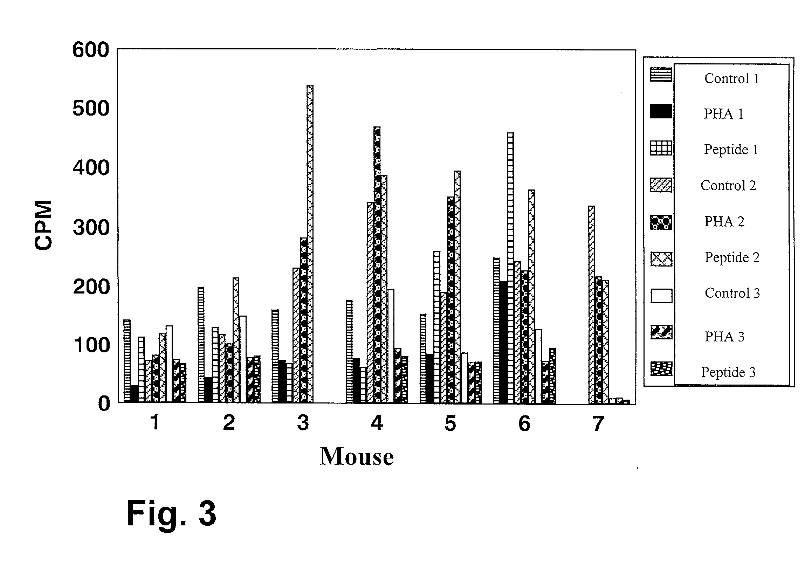

Figure 3 represents the response of proliferation for cells that are not stimulated (negative control), stimulated by PHA as a control for the proliferation of humans cells (because mouse cells do not respond in a significant way to this mitogen) and stimulated by the mixture of peptides after six days of *in vitro* stimulation.

We observed a lymphoproliferation against the mixture of peptides in an important number of mice. Due to the few number of cells used in these experiments, response is not so elevated but is higher than the response of donor's cells and naive mice. The responses were maximal at day 19 after immunisation and decreased at day 32.

### Detection of circulating human cells

The number of human CD2⁺ CD3⁺ cells in the peripheral blood of mice was detected by FACS (Table 1). The low number of circulating human cells may be the consequence of their sequestration in the lymphatic nodes. This phenomenon may be amplified by the successive immunisations and, in turns, may explain the decrease of the proliferative response after the third immunisation.

**Table 1:**

| | | | | |
|---|---|---|---|---|
| **Percentage of CD2**^{**+**} **CD3**^{**+**} **cells in NIH-III mice, implanted with hu-PBMC** | | | | |

| **Mouse** | **Day10** | **Day 20** | **Day 30** | **Day 41** |
|---|---|---|---|---|
| **1** | 18.03 | 19.1 | 43.4 | n.a. |
| **2** | 0.742 | 2.25 | 3.66 | n.a. |
| **3** | 30.3 | 58.49 | 78.4 | 96.52 |
| **4** | 0.68 | 8.89 | 1.48 | 0.86 |

CD2+ CD3 cells have been detected in the peripheric blood of four mice, at different days after the injection. Detection was realized by FACS with an antibody anti-CD2 coupled to phycoerythrin and with an antibody anti-CD3 coupled to fluorescein.
n.a.: not available

### Production of human immunoglobulins (hu-lg)

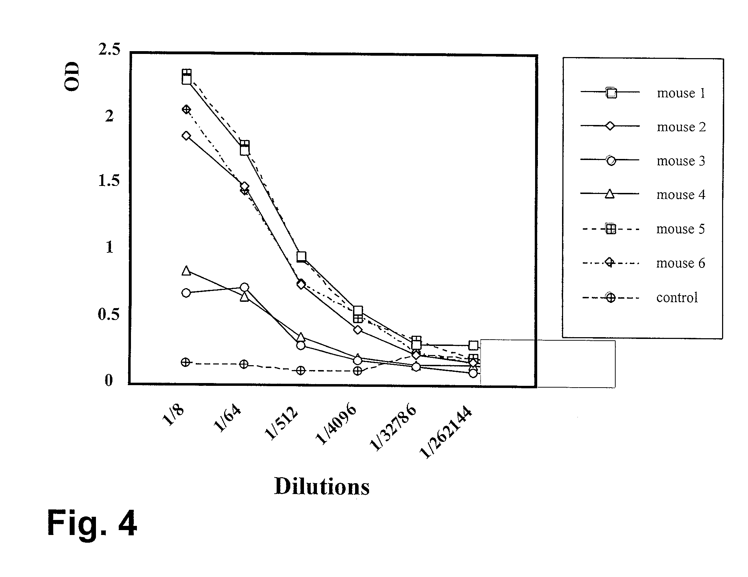

A strong production of hu-lg in the plasma of all mice grafted was detected using ELISA technique (Figure 4). This immunoglobulin production was detectable as early as day 7, after the graft.

### Production of specific immunoglobulins

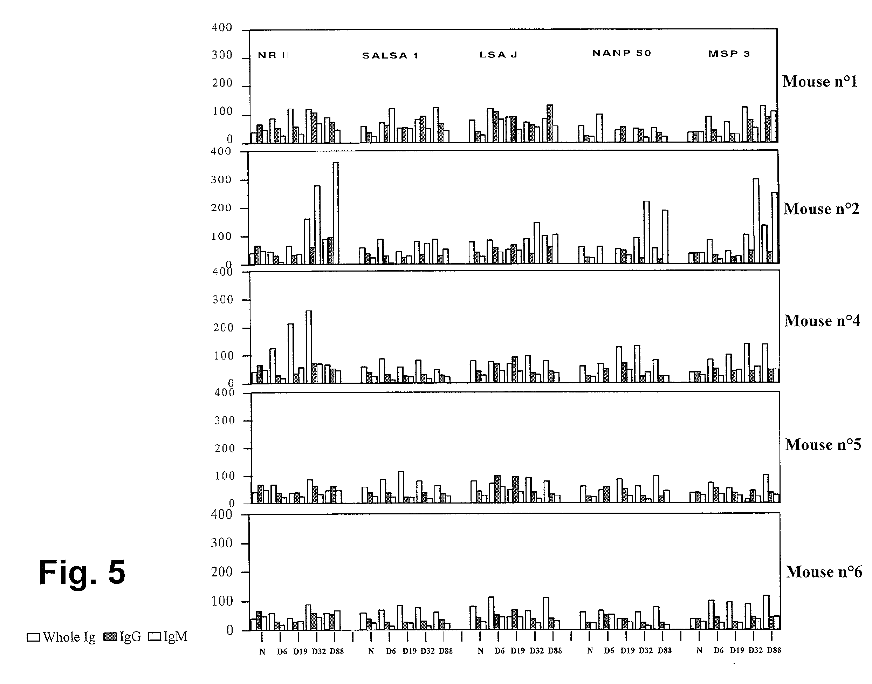

The specificity of the hu-lg was tested against each peptide for which the mice were immunised with. We found, in all mice, antibodies specific against the three lipopeptides. However, there is an important variability between mice in the lipopeptide response, even in a group receiving the same protocol of immunisation (Figure 5).

### Classes of hu-lg detected

We looked for the presence of human whole-lg in the plasma of the humanised mice. In the mice having the higher response to lipopeptides (mice N°2 and N°4), we detected the presence of lgM (mouse N°2) and maybe lgA (mouse N° 4) (Figure 5). Interestingly, the isotypic profile of secreted humans immunoglobulins seems to be function of the protocol of mice humanisation. It is possible that the environment, in which the cells are, at the moment of being re-stimulated by the antigen, may have an influence on the antibody classes produced.

Indeed, the cells grafted in the peritoneal cavity migrate to nodes. In the lymphatic nodes, the cells are stimulated by the parenchymateous cells and by the local cytokines to produce a humoral response, mainly constituted of lgM. In contrast, the cells grafted on collagen sponges stay in the peritoneum, which is a seric membrane, closed to a mucous membrane. Then, the peritoneal liquid and/or the cytokines released during the inflammatory process leading to vascularisation of collagen sponges may induce the production of lgA by the cells.

These results obtained with lymphocytes contribute to demonstrate the overall value of the model for the survival, replication and specific immune function of yet another human cell type in our model. They also contribute to establish the interest of the collagen matrix to generate neo-organs in which only human cells are present, from which they can be easily recovered and thereafter studied under *in vitro* conditions or, alternatively, used to graft other mice.

## Claims

1. A method of making a non-human mammal model comprising:
a. implanting, into an immunocompromised non-human mammal host, heterologous nucleated cells previously bound to a biocompatible support,
b. controlling non-adaptive defences of the non-human mammal host,
c. recovering a non-human mammal model harbouring settled heterologous nucleated cells capable of maintaining, differentiating and growing.

2. A method according to claim 1, wherein the steps of implanting said heterologous nucleated cells settled on a biocompatible support and controlling non-adaptive defences are inverted.

3. A method according to anyone of claims 1 to 2, wherein heterologous nucleated cells are bound to a support with a collagen gel.

4. A method according to anyone of claims 1 to 3, wherein the support comprising the heterologous nucleated cells settled thereon is implanted into the peritoneal cavity of the immunocompromised host.

5. A method according to anyone of claims 1 to 4, wherein said heterologous nucleated cells are cells that can be derived from a donor organ or from a partial resection, from healthy tissues surrounding a tumour, from a cell culture or from a tissue matrix.

6. A method according to anyone of claims 1 to 5, wherein the heterologous nucleated cells are non-infected, non-tumoral cells.

7. A method according to anyone of claims 1 to 6, wherein the control of non-adaptive defences encompasses a reduction of macrophages/monocytes and/or polymorphonuclear neutrophils (PMN) or a reduction of at least two of macrophages, monocytes or PMN cells, as measured by FACS analysis.

8. A method according to anyone of claims 1 to 7, wherein macrophages are depleted by controlled administration of a toxic compound in the non-human mammal host.

9. A method according to claim 8, wherein the administered toxic compound for macrophage depletion is the dichloromethylene diphosphonate (Cl₂MDP).

10. A method according to anyone of claims 8 to 9, wherein the toxic compound is contained in liposomes, which are injected in the non-human mammal host.

11. A method according to claim 10, wherein Cl₂MDP containing liposomes are injected at 4 day interval, starting two days after implanting the heterologous nucleated cells.

12. A method according to anyone of claims 1 to 11, wherein polymorphonuclear neutrophils (PMN) are depleted by administering PMN antagonists.

13. A method according to anyone of claims 1 to 12, wherein PMN are depleted by administering anti-PMN antibodies.

14. A method according to claim 13, wherein the anti-PMN antibodies are injected every 3-4 days, starting two days after implanting the heterologous nucleated cells.

15. A method according to anyone of claims 1 to 14, wherein the immunocompromised non-human mammal host is a rodent, preferably a mouse.

16. A method according to claim 15, wherein the immunocompromised mouse host is selected from the group consisting of:
- SCID mouse (severe combined immunodeficiency),
- SCID/Nod mouse (severe combined immunodeficiency/non obese diabetic),
- BXN (NIHIII or Beige Xid Nude) mouse,
- RAG mouse,
- RAG2 mouse,
- RAG-γC mouse.

17. A method according to any one of claims 1 to 16, wherein said heterologous nucleated cells are human hepatocytes.

18. A method according to any one of claims 1 to 16, wherein said heterologous nucleated cells are human lymphocytes

19. A non-human mammal model which is an immunocompromised non-human mammal host implanted with a support comprising heterologous nucleated cells settled thereon, and which non-adaptive defences are controlled to enable the heterologous nucleated cells of said implanted support to maintain, differentiate and grow.

20. A non-human mammal model according to claim 19, wherein said settled heterologous nucleated cells secrete specific proteins for several months.

21. A non-human mammal model according to anyone of claims 19 to 20, wherein said settled heterologous nucleated cells are receptive to pathogens having a restricted tropism therefore.

22. A non-human mammal model according to anyone of claims 19 to 21, wherein said settled heterologous nucleated cells are human hepatocytes.

23. A non-human mammal model according to claim 22, wherein said settled human hepatocytes are receptive to hepatotropic pathogens.

24. A non-human mammal model according to anyone of claims 19 to 21, wherein said settled heterologous nucleated cells are human lymphocytes.

25. A non-human mammal model model according to claim 24, wherein, after immunisation with antigens, said lymphocytes produce human lgG antibody and elicit lymphoproliferative responses, specific for said antigens.

26. A non-human mammal model according to claim 25, wherein the antigens are proteins derived from a hepatotropic pathogen.

27. A non-human mammal model according to claim 23 or 26, wherein hepatotropic pathogens are *Plasmodium* strains, HBV or HCV.

28. A non-human mammal model according to anyone of claims 19 to 27, wherein the non-human mammal immunocompromised host is a mouse.

29. A non-human mammal model according to claim 28, wherein the immunocompromised mouse host is selected from the group consisting of:
- SCID mouse (severe combined immunodeficiency),
- SCID/Nod mouse (severe combined immunodeficiency/non obese diabetic),
- BXN (NIHIII or Beige Xid Nude) mouse,
- RAG mouse,
- RAG2 mouse,
- RAG-γC mouse.

30. A tissue matrix comprising set of settled heterologous nucleated cells isolated from a non-human mammal model according to anyone of claims 19 to 29.

31. A tissue matrix according to claim 30, wherein said settled heterologous nucleated cells appear as individualized cells in suspension or in "solid" preparations.

32. A tissue matrix according to claim 30 wherein said settled heterologous nucleated cells appear as a cell line.

33. A method for studying a pathogen, in a non-human mammal model according to anyone of claims 19 to 29 comprising:
a. infecting said non-human mammal model with a pathogen, in conditions enabling said pathogen to enter in contact with the settled heterologous nucleated cells of the non-human mammal model
b. observing the pathogen-generated infection in said settled cells.

34. A method according to claim 33, wherein the infection is observed by light microscopy, by immunofluorescence antibody test (IFAT) using pathogen specific antibodies or by RT-PCR using primers for a pathogen specific gene.

35. A method according to anyone of claims 33 to 34, wherein the number of pathogens calculated by light microscopy, the staining obtained by IFAT and the transcripts detected in the settled human nucleated cells are compared to those of a control non-human mammal, infected by the same pathogen but devoid of cell implantation.

36. A method according to anyone of claims 33 to 35, wherein settled cells are human hepatocytes.

37. A method according to claim 36, wherein pathogens administrated to the mouse model are hepatotropic pathogens,

38. A method according to claim 37, wherein the hepatotropic pathogen is chosen among *Plasmodium* strains, HBV or HCV.

39. A method according to claim 38, wherein *Plasmodium falciparum* is used for infection and infection is observed by antibodies, specific of *Plasmodium falciparum* liver forms, or by nucleotide sequence amplification using primers specific for *Plasmodium falciparum* liver form genes.

40. A method according to claim 39, wherein said specific antibodies recognize the LSA-1 protein, or wherein said primers enable amplification of a sequence of the LSA1 gene.

41. A method according to anyone of claims 33 to 40, wherein the non-human mammal model is a mouse.

42. Use of a non-human mammal model according to claims 19 to 29 for the testing of a compound for a potential therapeutic interest.

43. A method for screening active compounds against the infection by a pathogen or against its detrimental effects in a non-human mammal model according to anyone of claims 19 to 29 comprising:
a. infecting said non-human mammal model with a pathogen, in conditions enabling said pathogen to penetrate the settled heterologous nucleated cells of the non-human mammal model,
b. administering the tested compound in conditions allowing its activity to occur,
c. observing the effects of said compound on the pathogen-generated infection or on its detrimental effects.

44. A method according to claim 43, wherein the infection is observed by light microscopy, by immunofluorescence antibody test (IFAT) using pathogen specific antibodies or by RT-PCR using primers for a pathogen specific gene.

45. A method according to anyone of claims 43 to 44, wherein the number of pathogens calculated by light microscopy, the staining obtained by IFAT and the transcripts detected in the settled cells are compared in the same non-human mammal model at different time points.

46. A method according to anyone of claims 43 to 45, wherein the pathogen administrated to the mouse model are hepatotropic pathogens.

47. A method according to claim 46, wherein the hepatotropic pathogen is chosen among *Plasmodium* strains, HBV or HCV.

48. A method according to claim 47, wherein *Plasmodium falciparum* is used for infection and infection is observed by antibodies, specific of *Plasmodium falciparum* liver forms, or by nucleotide sequence amplification using primers specific for *Plasmodium falciparum* liver form genes.

49. A method according to claim 48, wherein said specific antibodies recognize the LSA-1 protein, or wherein said primers enable amplification of a sequence of the LSA1 gene.

50. A method according to any one of claims 43 to 49, wherein the mammal non-human model is a mouse.

51. A method for screening the *in vivo* metabolism of xenobiotic compounds, in a non-human mammal model according to any one of claims 19 to 29 comprising:
a. administrating the xenobiotic compound to be tested to said non-human mammal model in conditions allowing the compound to interact with settled heterologous nucleated cells,
b. observing its biotransformation at the level of said settled cells.

52. A method according to claim 51, wherein the compound biotranformation is evaluated by the detection and/or measurement of the level of metabolites produced by said settled cells prior and after administration of the compound.

53. A method according to anyone of claims 51 to 52, wherein toxic effects on human nucleated cells are evaluated and where the appropriate compound doses, at which the effects appear, calculated.

54. A method according to anyone of claims 51 to 53, wherein potential interactions between reactive metabolites and cellular macromolecules are studied.

55. A method according to anyone of claims 51 to 54, wherein heterologous nucleated cells are human hepatocytes.

56. A technical platform, useful to identify new compounds useful to treat mammal infections provoked by a pathogen, **characterized in that** it comprises at least a chimeric model according to anyone of claims 19 to 29 and appropriate means to detect or to observe the effects of said compounds on a pathogen-generated infection of said model.

57. A technical platform, useful for screening the *in vivo* metabolism of xenobiotic compounds **characterized in that** it comprises at least a chimeric model according to anyone of claims 19 to 29 and appropriate means to observe the biotransformation of said compounds by implanted human cells in said model.
